# EUROPEAN PATENT APPLICATION

(11) **EP 4 675 891 A1**
(43) Date of publication of application: **07.01.2026**
(21) Application number: 25186203.3
(22) Date of filing: 30.06.2025
(51) Int. Cl.: H02J 50/00, H04B 5/79, A61B 5/00, H10N 10/00, H10N 15/00

(54) **POWER MANAGEMENT UNIT WITH NFC-BASED COLD-START FOR BATTERYLESS DEVICE POWERED BY THERMOELECTRIC ENERGY HARVESTER**

(30) Priority: 04.07.2024 IN 202441051303
(71) Applicant: NXP B.V., 5656 AG Eindhoven (NL)
(72) Inventor: Ghosh, Jyotirmoy, 5656AG Eindhoven (NL); Kokkatu, Abhilash Muraleedharan, 5656AG Eindhoven (NL); Pigott, John, 5656AG Eindhoven (NL)
(74) Representative: Miles, John Richard

(57) **Abstract**

Embodiments of a power management unit (100) for a device, a power management unit (100) for a biomedical patch, and a biomedical patch are disclosed. In an embodiment, a power management unit (100) for a batteryless device includes a Thermoelectric Energy Generator, TEG, harvester (108), a Near Field Communication, NFC, harvester (106), a DC-DC converter (104) coupled between the TEG harvester (108) and the NFC harvester (106), a first switch (S1) and a second switch (S2) coupled between the DC-DC converter (104) and the NFC harvester (106), a central control unit, CCU, (102) coupled between the first switch (S1) and the second switch (S2) and configured to control the DC-DC converter (104), and a capacitor (114) coupled between the CCU (102), the first (S1) and second switches (S2), and a load (118). Other embodiments are also disclosed.

## Description

### BACKGROUND

With the recent advancements in semiconductor fabrication technology, Thermoelectric Energy Generators (TEGs) can be used as an alternative to batteries, for example, for connected bio-medical, for personal health devices, and for sustainable and green solutions. However, based on the temperature difference between two electrodes, a TEG's output voltage can vary and may be too low to start-up a device or continuously supply a fixed load. Therefore, there is a need for an efficient power management unit for a TEG powered biomedical device that can operate under a wide range of TEG output voltage.

### SUMMARY

Embodiments of a power management unit for a batteryless device, a power management unit for a batteryless biomedical patch, and a batteryless biomedical patch are disclosed. In an embodiment, a power management unit for a batteryless device includes a Thermoelectric Energy Generator (TEG) harvester, a Near Field Communication (NFC) harvester, a Direct Current (DC)-DC converter coupled between the TEG harvester and the NFC harvester, a first switch and a second switch coupled between the DC-DC converter and the NFC harvester, a central control unit (CCU) coupled between the first switch and the second switch and configured to control the DC-DC converter, and a capacitor coupled between the CCU, the first and second switches, and a load. Other embodiments are also disclosed.

In an embodiment, the NFC harvester is further configured to charge the capacitor through the second switch during a start-up event to activate the CCU.

In an embodiment, the second switch includes a unidirectional switch.

In an embodiment, the NFC harvester is further configured to charge the capacitor through the second switch to supply the load with an energy from the NFC harvester.

In an embodiment, the CCU is further configured to enable the DC-DC converter to start boosting an output voltage from the TEG harvester.

In an embodiment, when the output voltage from the DC-DC converter reaches a voltage level between the first switch and the second switch, the first switch is turned on and the load is supplied with an energy from the TEG harvester.

In an embodiment, the power management unit further includes a shunt clamp coupled in parallel with the capacitor and the load.

In an embodiment, the power management unit further includes a third switch coupled between the capacitor and the load.

In an embodiment, the power management unit further includes a rectifier coupled between the TEG harvester and the DC-DC converter.

In an embodiment, the power management unit further includes a second capacitor coupled between the TEG harvester and the DC-DC converter.

In an embodiment, the power management unit does not include a battery.

In an embodiment, the power management unit includes the load.

In an embodiment, a power management unit for a batteryless biomedical patch includes a TEG harvester, an NFC harvester, a DC-DC converter coupled between the TEG harvester and the NFC harvester, a first switch and a second switch coupled between the DC-DC converter and the NFC harvester, a CCU coupled between the first switch and the second switch and configured to control the DC-DC converter, a capacitor coupled between the CCU, the first and second switches, and a load, a third switch coupled between the capacitor and the load, and a shunt clamp coupled in parallel with the capacitor and the load.

In an embodiment, the NFC harvester is further configured to charge the capacitor through the second switch during a start-up event to activate the CCU, and wherein the second switch comprises a unidirectional switch.

In an embodiment, the NFC harvester is further configured to charge the capacitor through the second switch to supply the load with an energy from the NFC harvester.

In an embodiment, the CCU is further configured to enable the DC-DC converter to start boosting an output voltage from the TEG harvester.

In an embodiment, when the output voltage from the DC-DC converter reaches a voltage level between the first switch and the second switch, the first switch is turned on and the load is supplied with an energy from the TEG harvester.

In an embodiment, the power management unit further includes a rectifier coupled between the TEG harvester and the DC-DC converter.

In an embodiment, the power management unit does not include a battery.

In an embodiment, a batteryless biomedical patch includes a biomedical circuit and a power management unit, which includes a TEG harvester, an NFC harvester, a DC-DC converter coupled between the TEG harvester and the NFC harvester, a first switch and a second switch coupled between the DC-DC converter and the NFC harvester, a CCU coupled between the first switch and the second switch and configured to control the DC-DC converter, and a capacitor coupled between the CCU, the first and second switches, and the biomedical circuit.

Other aspects in accordance with the invention will become apparent from the following detailed description, taken in conjunction with the accompanying drawings, illustrated by way of example of the principles of the invention.

### BRIEF DESCRIPTION OF THE DRAWINGS

Fig. 1 depicts a power management unit in accordance with an embodiment of the invention.
Fig. 2 is a flow chart that illustrates an exemplary system start-up sequence and load management operation that can be performed by the power management unit depicted in Fig. 1.
Fig. 3 illustrates a signal timing diagram of the power management unit depicted in Fig. 1.
Fig. 4 illustrates another signal timing diagram of the power management unit depicted in Fig. 1.
Fig. 5 illustrates another signal timing diagram of the power management unit depicted in Fig. 1.
Fig. 6 depicts a person having a batteryless biomedical patch that contains the power management unit depicted in Fig. 1.

Throughout the description, similar reference numbers may be used to identify similar elements.

### DETAILED DESCRIPTION

It will be readily understood that the components of the embodiments as generally described herein and illustrated in the appended figures could be arranged and designed in a wide variety of different configurations. Thus, the following more detailed description of various embodiments, as represented in the figures, is not intended to limit the scope of the present disclosure, but is merely representative of various embodiments. While the various aspects of the embodiments are presented in drawings, the drawings are not necessarily drawn to scale unless specifically indicated.

The present invention may be embodied in other specific forms without departing from its spirit or essential characteristics. The described embodiments are to be considered in all respects only as illustrative and not restrictive. The scope of the invention is, therefore, indicated by the appended claims rather than by this detailed description. All changes which come within the meaning and range of equivalency of the claims are to be embraced within their scope.

Reference throughout this specification to features, advantages, or similar language does not imply that all of the features and advantages that may be realized with the present invention should be or are in any single embodiment of the invention. Rather, language referring to the features and advantages is understood to mean that a specific feature, advantage, or characteristic described in connection with an embodiment is included in at least one embodiment of the present invention. Thus, discussions of the features and advantages, and similar language, throughout this specification may, but do not necessarily, refer to the same embodiment.

Furthermore, the described features, advantages, and characteristics of the invention may be combined in any suitable manner in one or more embodiments. One skilled in the relevant art will recognize, in light of the description herein, that the invention can be practiced without one or more of the specific features or advantages of a particular embodiment. In other instances, additional features and advantages may be recognized in certain embodiments that may not be present in all embodiments of the invention.

Reference throughout this specification to "one embodiment," "an embodiment," or similar language means that a particular feature, structure, or characteristic described in connection with the indicated embodiment is included in at least one embodiment of the present invention. Thus, the phrases "in one embodiment," "in an embodiment," and similar language throughout this specification may, but do not necessarily, all refer to the same embodiment.

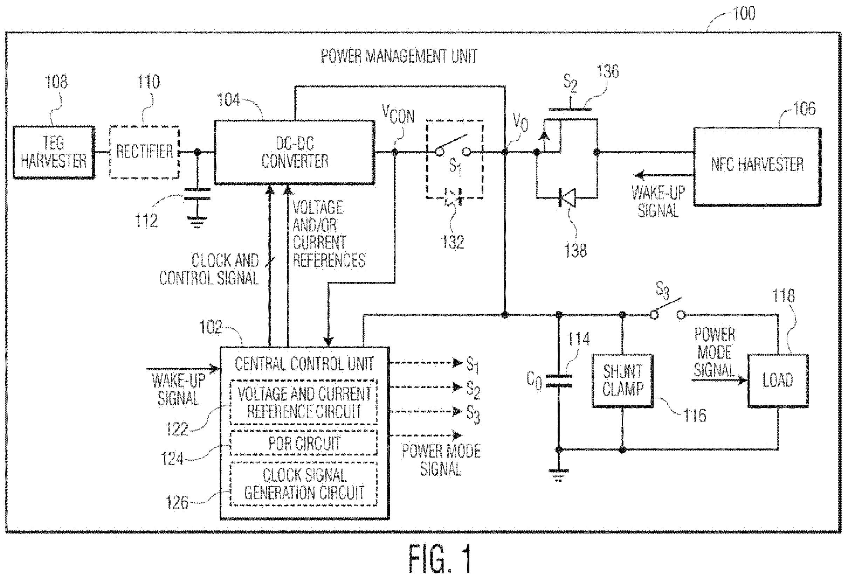

Fig. 1 depicts a power management unit 100 in accordance with an embodiment of the invention. In the embodiment depicted in Fig. 1, the power management unit 100 includes a central control unit (CCU) 102, a DC-DC converter 104, a Near Field Communication (NFC) harvester 106, a TEG harvester 108, an optional rectifier 110, three switches *S₁, S₂, S₃,* two capacitors 112, 114, a shunt clamp 116, and a load 118. In the embodiment depicted in Fig. 1, the DC-DC converter 104 is coupled between the TEG harvester 108 and the NFC harvester 106. The switches *S₁, S₂* are coupled between the DC-DC converter 104 and the NFC harvester 106. The CCU 102 is coupled between the switch *S₁* and the switch *S₂* and configured to control the DC-DC converter 104. The capacitor 114, which is also referred to as the bootstrap capacitor, is coupled between the CCU 102, the switches *S₁, S₂,* and the load 118. The power management unit 100 can be used in various applications, such as medical applications, computer applications, and/or consumer or appliance applications. In some embodiments, at least one or some of the components of the power management unit 100 is/are implemented in a substrate and is/are packaged as a stand-alone semiconductor integrated circuit (IC) device or chip. In some embodiments, at least one or some of the components of power management unit 100 is/are implemented in a substrate, such as a semiconductor wafer or a printed circuit board (PCB). In some embodiments, the power management unit 100 is included in a batteryless device (i.e., a circuit that does not include any battery) and the power management unit 100 also does not include any battery. Although the depicted power management unit 100 is shown in Fig. 1 with certain components and described with certain functionality herein, other embodiments of the power management unit 100 may include fewer or more components to implement the same, less, or more functionality. For example, although the power management unit 100 is shown in Fig. 1 includes the load 118, in other embodiments, the load 118 is external to the power management unit 100 and is not included in the power management unit 100. In some embodiments, the power management unit 100 is included in a batteryless device (i.e., a circuit that does not include any battery) and the load is a circuit of the batteryless device that receives power from the power management unit. For example, the power management unit 100 is included in a batteryless biomedical patch that does not include any battery and the load is a biomedical circuit of the batteryless biomedical patch that receives power from the power management unit. In another example, although the power management unit 100 is shown in Fig. 1 as being connected in a certain topology, the network topology of the power management unit 100 is not limited to the topology shown in Fig. 1. In some embodiments, a first element is coupled to or connected to a second element in a direct connection between the first element and the second element and/or an indirect connection between the first element and the second element. In some embodiments, a first element is coupled to or connected to a second element through a direct or indirect connection, either physical or electrical, between the first element and the second element.

Bio-medical devices and health monitor patches, such as, cardiovascular signal monitors or blood glucose monitors are normally powered by low-capacity batteries. When depleted, these batteries must be recharged or replaced by new ones, which can pose serious problems. Firstly, in many applications where devices are placed at inaccessible areas or devices implanted in human bodies, it is impractical to replace or recharge the batteries. Secondly, longer shelf-life of the devices results significant loss in battery capacity due to leakage. Thirdly, improper disposal of batteries creates environmental pollutions. With advancements in semiconductor fabrication technology, an alternative solution is to use miniaturized energy harvesters, which can extract energy from ambient to a usable form for these applications. For example, Thermoelectric Energy Generators (TEGs), or TEG harvesters, can be used as battery alternatives as these harvesters can continuously generate electrical energy from the difference between the human body temperature and the ambient temperature. TEGs are suitable for integration into bulk and flexible devices, such as, health patches, wrist bands, thermal vests, etc. Furthermore, as TEGs are static harvesters, they do not require any body movement to generate energy and can be placed at a stationary part of human body. Despite the advantages mentioned above, TEGs have one significant disadvantage. Because the harvested energy by a TEG is a function of the difference between the human body temperature and the ambient temperature, the output potential can vary and may be very low, depending on the temperature difference. For instance, the open circuit voltage of a TEG, even with very efficient thermoelectric material, can be in the order of 5 millivolt (mV) - 20mV for a temperature difference of 1degK. While a step-up dc-dc voltage converter (e.g., the DC-DC converter 104) can generate a higher voltage from the harvester output, a circuit cannot start-up at such low potential or continuously provide sufficient energy required by the load. Consequently, a TEG system requires a specialized power management unit (e.g., the power management unit 100) that is adaptive to the TEG output with dedicated cold-start (e.g., when the voltage level is below a minimum level) and load management.

In the embodiment depicted in Fig. 1, the power management unit 100 adapts a start-up sequence by using NFC power from the NFC harvester 106 to wake-up the CCU 102 and to kick-start the DC-DC converter 104 and manage the load consumption (e.g., by adjusting the Bluetooth transmission interval), which can eliminate the need for a separate cold-start system for the TEG harvester 108. Consequently, the load 118 is only connected when the CCU 102 determines that sufficient (minimal) power is available. The load 118 is managed to only consume power less than the available power from either the NFC harvester 106 or the TEG harvester 108, e.g., by delaying transmissions or data collection, or using various hibernate modes.

In the embodiment depicted in Fig. 1, the CCU 102 acts as the core of the power management unit 100. In some embodiments, the CCU 102 includes at least one voltage and current reference circuit 122 configured to generate at least one reference voltage and/or current, a power-on reset (POR) circuit configured to generate a reset signal when power is applied to the CCU 102, and at least one clock signal generation circuit 126 configured to generate at least one clock signal. In some embodiments, the CCU is configured to enable the DC-DC converter to start boosting the output voltage from the TEG harvester. In some embodiments, the CCU is configured to control the switching frequency of the DC-DC converter 104 and perform the load management of the load 118. In some embodiments, the CCU 102 is configured to activate by a wake-up signal from the NFC harvester 106. In some embodiments, the CCU is configured to generate one or more control signals for one or more of the switches *S₁, S₂, S₃.* In some embodiments, the CCU 102 is configured to generate a power mode signal for controlling the power mode of the load. The CCU 102 may be implemented as hardware, software, firmware, and/or a combination of hardware, software, and/or firmware. In some embodiments, the CCU 102 is implemented using a processor, such as, a microcontroller or a CPU.

In the embodiment depicted in Fig. 1, the DC-DC converter 104 is configured to boost up the output voltage of the TEG harvester 108 to a voltage V_{CON} that is within a specified load voltage range. In some embodiments, the DC-DC converter is a boost DC-DC converter whose output voltage is higher than it input voltage. The DC-DC converter 104 may be a high conversion ratio step-up DC-DC converter with maximum power point tracking (MPPT), which is a technique used with variable power sources to maximize energy extraction as conditions vary. In some embodiments, while the output voltage of the DC-DC converter 104 is not regulated, an MPPT algorithm regulates the input impedance of the DC-DC converter 104 to extract maximum available power from the TEG harvester 108.

In the embodiment depicted in Fig. 1, the power management unit 100 has two energy sources, which are the NFC harvester 106 and the TEG harvester 108. The TEG harvester 108 operates as the main energy source. The primary function of the NFC harvester 106 is to assist the system start-up. In addition, the NFC harvester 106 can also supply the load 118 initially during activation/cold-start. Both the NFC harvester 106 and the TEG harvester 108 transfer energy to the capacitor 114, which operates as a charge storage element with a capacitance value of *C_{O},* and filters all circuitry of the power management unit 100 including the load 118. Consequently, the capacitor 114 can be used to ensure maximum utilization of energy from both the NFC harvester 106 and the TEG harvester 108.

In some embodiments, the switches *S₁, S₂* are unidirectional switches that are only conductive in one particular signal direction. For example, the switch *S₁* is implemented as a diode 132 and the switch *S₂* is implemented as a transistor (e.g., a metal-oxide-semiconductor field-effect transistor (MOSFET)) 136 with a diode 138. However, the switches *S₁, S₂* may be implemented differently from the embodiments depicted in Fig. 1. In the embodiment depicted in Fig. 1, the NFC harvester 106 and the TEG harvester 108 are coupled to the capacitor 114 through the unidirectional switches *S₁* and *S₂* to block any reverse flow of current from the capacitor 114 to the respective harvester, while the switch *S₃* is coupled between the capacitor 114 and the load 118 and may be controlled by the CCU 102 to connect or disconnect the load 118 based on the voltage level *V_{O}* of the capacitor 114. In some embodiments, the NFC harvester 106 is configured to charge the capacitor 114 through the switch *S₂* during a start-up event (e.g., a cold-start) to activate the CCU 102. In some embodiments, the NFC harvester 106 is configured to charge the capacitor 114 through the switch *S₂* to supply the load 118 with the energy from the NFC harvester 106. In some embodiments, when the output voltage V_{CON} from the DC-DC converter 104 reaches the voltage level *V_{O}* between the switches *S₁* and *S₂*, the switch *S₁* is turned on (e.g., being conductive) and the load 118 is supplied with the energy from the TEG harvester 108.

In the embodiment depicted in Fig. 1, the TEG harvester 108 can generate power depending on the temperature difference on two plates. In some embodiments, the TEG harvester 108 continuously generates electrical energy from the difference between the human body temperature and the ambient temperature. As used herein a TEG, or TEG harvester, may include any device, devices, and/or circuit that generates electrical energy from a difference between at least two temperatures. The optional rectifier 110 is coupled between the TEG harvester 108 and the DC-DC converter 104. In some embodiments, the rectifier 110 is coupled to the output of the TEG harvester 108 for reverse temperature gradient control. The capacitor 112 is coupled between the TEG harvester 108 and the DC-DC converter 104 and may be connected to a fixed voltage (e.g., the ground).

In the embodiment depicted in Fig. 1, the shunt clamp 116 is coupled in parallel with the capacitor 114 and the load 118. In some embodiments, the shunt clamp 116 is a shunt voltage clamp and is connected across the capacitor 114 to define the highest limit *V_{O_MAX}* of the capacitor voltage *V_{O}.* Consequently, the shunt clamp 116 acts as a primary protection device and does not cause additional losses below *V_{O_MAX}.* Although the power management unit 100 is shown in Fig. 1 includes the shunt clamp 116, in other embodiments, the shunt clamp 116 is external to the power management unit 100 and are not included in the power management unit 100.

In an example operation of the power management unit 100, an efficient power management with cold-start using Near Field Communication (NFC) module for ThermoElectric Generator (TEG) powered batteryless health monitor patches is implemented. During the NFC-based cold-start, a single tap from an NFC activation device provide sufficient potential and energy to the NFC harvester 106 that can generate a distinct clock and a gate driver signal even when the TEG output voltage is less than the MOSFET Threshold voltage (*V_{TH}*) of the switch *S₂*. The NFC harvester 106, in turn, enables the DC-DC converter 104 to start boosting the output voltage from the TEG harvester 108. Because an NFC module is typically already present in most health patches for patch activation, the power management unit 100 does not require any additional devices or hardware for cold-start. In the adaptive load management operation, the CCU 102 tracks the bootstrap capacitor voltage of the capacitor 114 and defines the 'power mode' of the load 118. For example, if the TEG output voltage is low, the CCU can put the load 118 in a low-power mode by, for example, increasing the Bluetooth data transfer interval from a health patch containing the power management unit 100. On the other hand, when the TEG harvester 108 generates a higher output power, the transmission interval can be reduced subsequently in a high-power mode. In the power management operation, the high conversion ratio DC-DC step-up converter with Maximum Power Point Tracking (MPPT) 104 can regulate the input impedance of the DC-DC converter 104 to extract maximum power available from the TEG harvester 108. While the DC-DC converter 104 can regulate the input, the DC-DC converter 104 can operate without any feedback loop from the output, which is connected to the capacitor 114. Consequently, the DC-DC converter 104 can generate an output voltage based on the TEG output. The shunt clamp can provide protection for the load 118.

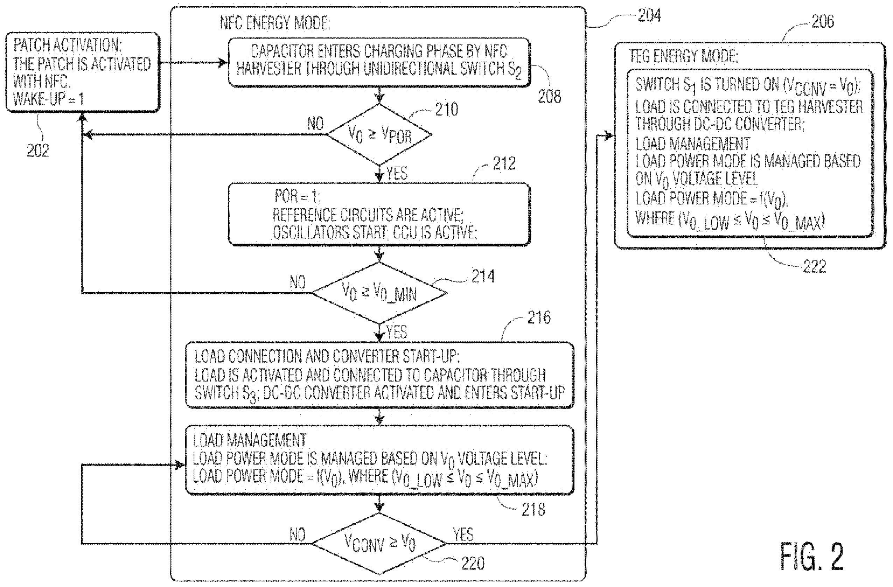

Fig. 2 is a flow chart that illustrates an exemplary system start-up sequence and load management operation that can be performed by the power management unit 100 depicted in Fig. 1. At step 202, the system start-up sequence and load management operation starts. The NFC harvester 106 can provide an excellent cold-start option to the DC-DC converter 104 boosting up the TEG output. As illustrated in Fig. 2, when a batteryless patch containing the power management unit 100 is tapped and activated ("woken-up") with an NFC module, the NFC harvester 106 can charge the capacitor 114 to a voltage level sufficient to start-up oscillators and reference generators circuits in the CCU 102 at step 208 under an NFC energy mode 204. It is determined whether or not *Vo* rises above *V_{O_POR}* at step 210. If/when *Vo* rises above *V_{O_POR}* , the POR is de-asserted, and logic circuits, oscillators and reference generators circuits in the CCU 102 are activated at step 212. Subsequently, it is determined whether or not *Vo* rises above *V_{O_MIN}* at step 214. At step 216, if/when *Vo* rises above *V_{O_MIN}* , the load switch *S₃* is turned on by the CCU 102 and the load 118 is supplied by NFC energy, and in parallel, the step-up DC-DC converter 104 also enters the start-up phase. The CCU 102 monitors the converter output voltage (*V_{CONV}*)*.* As the load 118 is activated, the CCU 102 starts managing load power consumption based on *Vo* level [*Load Power Mode* = *f*(*V_{O}*), where (*V_{O_MIN}* ≤ *V_{O}* ≤ *V_{O_MAX})*]. Subsequently, it is determined whether or not *V_{CONV}* rises above *Vo* at step 220. At step 222, if/when *V_{CONV}* crosses *Vo* level (V_{CONV} ≥ V_{O}), the CCU 102 tums on the switch *S₁* and the TEG harvester 108 starts supplying the load 118 via the DC-DC converter 104 and the CCU 102 starts managing load power consumption based on *Vo* level *[Load Power Mode = f(V_{O}),* where (*V_{O_MIN}* ≤ *Vo* ≤ *V_{O_MAX})]* under a TEG energy mode 206. For example, an NFC energy harvester in NHS7900 CGM health patch can generate *Voltage V_{EH,0} = 2.7V (Unloaded output voltage*), Current I_{EH,2.0}* = *8.0mA (For a typical output voltage of 2.0V**),* at a received field strength of 4.5 A/m and using class-6 antenna, for an unmodulated 13.5 MHz NFC signal.

For load management, the CCU 102 can track the capacitor voltage *Vo* of the capacitor 114 and define the 'power mode' of the load 118. For example, three intermediate voltage levels *(V_{O_MIN}, V_{O_LP}* and *V_{O_HP}* ) can be defined to determine the following power modes:
*Vo* ≤ *V_{O_MIN}* : the load 118 is disconnected;
*V_{O_MIN}* ≤ *Vo* ≤ *V_{O_LP}*: the load 118 is in Ultra-Low Power mode (ULP);
*V_{O_LP}* ≤ *Vo* ≤ *V_{O_HP}*: the load 118 is in Low Power mode (LP);
*Vo* ≥ *V_{O_HP}* : the load 118 is in High Power mode (HP).

There can be additional power modes inserted between these modes using intermediate threshold voltages.

For instance, after the start-up or during the runtime, if the output voltage *Vo* of the CCU 102 is low (VO_LP ≤ VO ≤ VO_HP ), the CCU 102 can put the load 118 in LP mode, for example, by increasing the Bluetooth data transfer interval from a health patch containing the power management unit 100. On the other hand, when the TEG harvester 108 or the NFC harvester 106 generates higher output voltage (VO ≥ VO_HP ), the Bluetooth connectivity interval can be reduced in HP mode. In general, the load 118 can operate at consumption levels that are aligned with the available power.

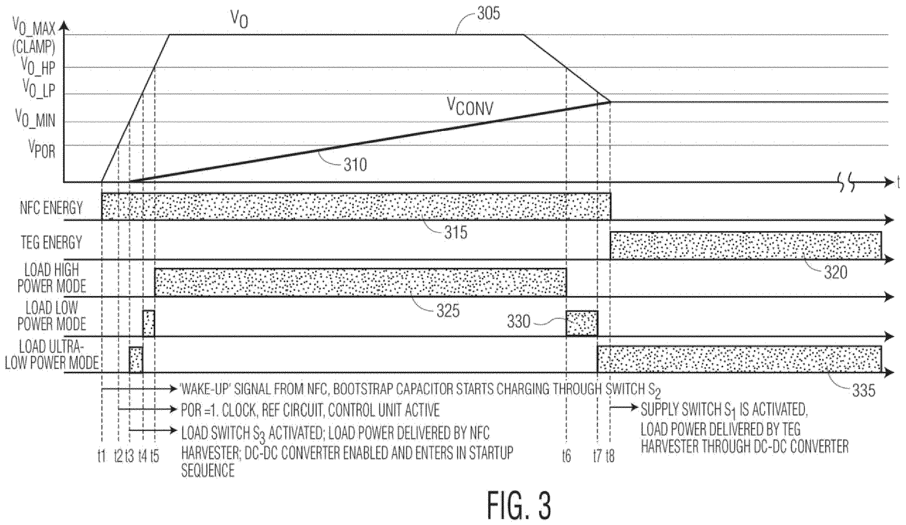

Fig. 3 illustrates a signal timing diagram of the power management unit 100 depicted in Fig. 1. In the signal timing diagram of Fig. 3, example waveforms 305, 310, 315, 320, 325, 330, 335 of voltage VO, voltage VCONV, NFC energy, TEG energy, a load high power mode signal, a load low power mode signal, a load ultra-low power mode signal of the power management unit 100 depicted in Fig. 1 are illustrated, respectively. At time point t1, a 'Wake-up' signal from an NFC module is applied and the capacitor 114 starts charging through the switch S2. At time point t2, the POR is asserted, and logic circuits, oscillators and reference generators circuits in the CCU 102 are activated. At time point t3, the load switch S3 is activated and load power is delivered by the NFC harvester 106, the DC-DC converter 104 is enabled and enters in startup sequence. At time point t4, the load 118 switches from the load ultra-low power mode to the load low power mode. At time point t5, the load 118 switches from the load low power mode to the load high power mode. At time point t6, the load 118 switches from the load high power mode to the load low power mode. At time point t7, the load 118 switches from the load low power mode to the load ultra-low power mode. Between time point t1 and time point t7, load power is delivered by the TEG harvester 108 (i.e., the NFC energy value 315 is positive (e.g., 1)). At time point t8, voltage VO and voltage VCONV converge at a level that is between VO_MIN and VO_LP because the load 118 is in Ultra-Low Power mode (ULP), the supply switch S1 is activated, and load power is delivered by the TEG harvester 108 through the DC-DC converter 104 (i.e., the TEG energy value 320 is positive (e.g., 1)).

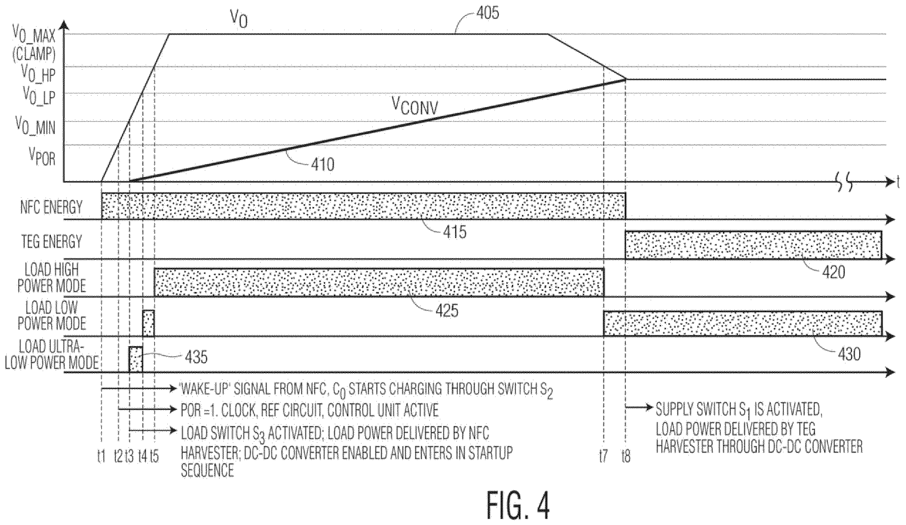

Fig. 4 illustrates another signal timing diagram of the power management unit 100 depicted in Fig. 1. In the signal timing diagram of Fig. 4, example waveforms 405, 410, 415, 420, 425, 430, 435 of voltage VO, voltage VCONV, NFC energy, TEG energy, a load high power mode signal, a load low power mode signal, a load ultra-low power mode signal of the power management unit 100 depicted in Fig. 1 are illustrated, respectively. At time point t1, a 'Wake-up' signal from an NFC module is applied and the capacitor 114 starts charging through the switch S2. At time point t2, the POR is asserted, and logic circuits, oscillators and reference generators circuits in the CCU 102 are activated. At time point t3, the load switch S3 is activated and load power is delivered by the NFC harvester 106, the DC-DC converter 104 is enabled and enters in startup sequence. At time point t4, the load 118 switches from the load ultra-low power mode to the load low power mode. At time point t5, the load 118 switches from the load low power mode to the load high power mode. At time point t7, the load 118 switches from the load high power mode to the load low power mode. Between time point t1 and time point t7, load power is delivered by the TEG harvester 108 (i.e., the NFC energy value 415 is positive (e.g., 1)). At time point t8, voltage VO and voltage VCONV converge at a level that is between VO_LP and VO_HP because the load 118 is in Low Power mode (LP), the supply switch S1 is activated, and load power is delivered by the TEG harvester 108 through the DC-DC converter 104 (i.e., the TEG energy value 420 is positive (e.g., 1)).

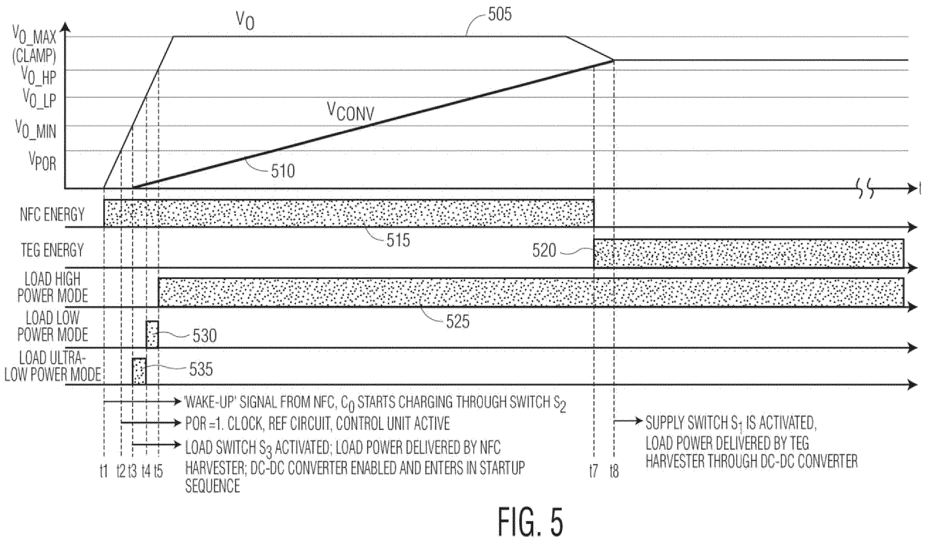

Fig. 5 illustrates a signal timing diagram of the power management unit 100 depicted in Fig. 1. In the signal timing diagram of Fig. 5, example waveforms 505, 510, 515, 520, 525, 530, 535 of voltage VO, voltage VCONV, NFC energy, TEG energy, a load high power mode signal, a load low power mode signal, a load ultra-low power mode signal of the power management unit 100 depicted in Fig. 1 are illustrated, respectively. At time point t1, a 'Wake-up' signal from an NFC module is applied and the capacitor 114 starts charging through the switch S2. At time point t2, the POR is asserted, and logic circuits, oscillators and reference generators circuits in the CCU 102 are activated. At time point t3, the load switch S3 is activated and load power is delivered by the NFC harvester 106, the DC-DC converter 104 is enabled and enters in startup sequence. At time point t4, the load 118 switches from the load ultra-low power mode to the load low power mode. At time point t5, the load 118 switches from the load low power mode to the load high power mode. Between time point t1 and time point t7, load power is delivered by the TEG harvester 108 (i.e., the NFC energy value 515 is positive (e.g., 1)). At time point t8, voltage VO and voltage VCONV converge at a level that is higher than VO_HP because the load 118 is in High Power mode (HP), the supply switch S1 is activated, and load power is delivered by the TEG harvester 108 through the DC-DC converter 104 (i.e., the TEG energy value 520 is positive (e.g., 1)).

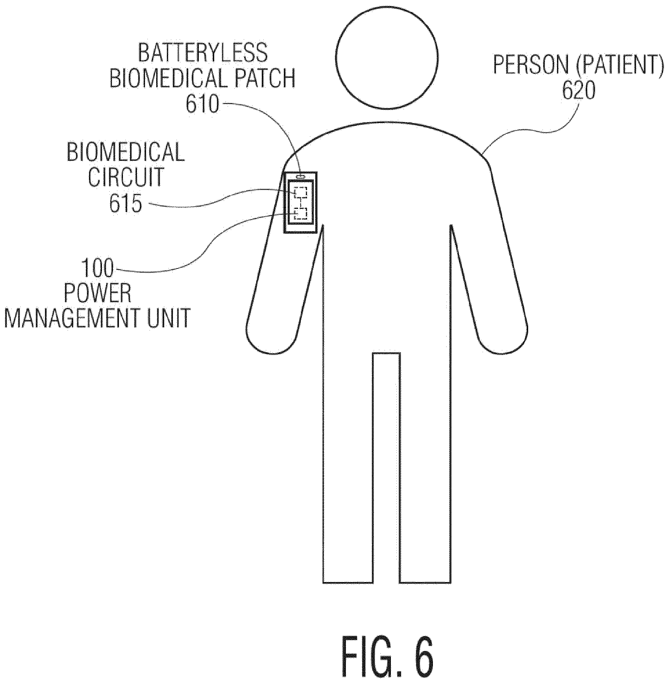

Fig. 6 depicts a person (e.g., a patient) 620 having a batteryless biomedical patch 610 that contains the power management unit 100 depicted in Fig. 1. In the embodiment depicted in Fig. 6, the batteryless biomedical patch 610 is embedded into the body (e.g., under the skin) of the person (e.g., a patient) 620, operating based on power from the power management unit 100 depicted in Fig. 1. The batteryless biomedical patch 610 can be used to monitor a characteristic (e.g., the glucose level or the blood pressure) of the person (e.g., a patient) 620. For example, the batteryless biomedical patch 610 may include a biomedical circuit 615 configured to monitor a characteristic (e.g., the glucose level or the blood pressure) of the person (e.g., a patient) 620 and the power management unit 100 configured to supply power to the biomedical circuit 615 using NFC energy or TEG energy and to perform load management for the biomedical circuit 615.

Embodiments of a power management unit for a device, a power management unit for a biomedical patch, and a biomedical patch are disclosed. In an embodiment, a power management unit for a batteryless device includes a Thermoelectric Energy Generator (TEG) harvester, a Near Field Communication (NFC) harvester, a Direct Current (DC)-DC converter coupled between the TEG harvester and the NFC harvester, a first switch and a second switch coupled between the DC-DC converter and the NFC harvester, a central control unit (CCU) coupled between the first switch and the second switch and configured to control the DC-DC converter, and a capacitor coupled between the CCU, the first and second switches, and a load. Other embodiments are also disclosed.

It should be noted that at least some of the operations described herein may be implemented using software instructions stored on a computer useable storage medium for execution by a computer. As an example, an embodiment of a computer program product includes a computer useable storage medium to store a computer readable program.

Alternatively, embodiments of the invention may be implemented entirely in hardware or in an implementation containing both hardware and software elements. In embodiments which use software, the software may include but is not limited to firmware, resident software, microcode, etc.

Although specific embodiments of the invention have been described and illustrated, the invention is not to be limited to the specific forms or arrangements of parts so described and illustrated. The scope of the invention is to be defined by the claims appended hereto and their equivalents.

## Claims

1. A power management unit comprising:
a Thermoelectric Energy Generator (TEG) harvester;
a Near Field Communication (NFC) harvester;
a Direct Current (DC)-DC converter coupled between the TEG harvester and the NFC harvester;
a first switch and a second switch coupled between the DC-DC converter and the NFC harvester;
a central control unit (CCU) coupled between the first switch and the second switch and configured to control the DC-DC converter; and
a capacitor coupled between the CCU, the first and second switches, and a load.

2. The power management unit of claim 1, wherein the NFC harvester is further configured to charge the capacitor through the second switch during a start-up event to activate the CCU.

3. The power management unit of claim 2, wherein the second switch comprises a unidirectional switch.

4. The power management unit of claim 2 or 3, wherein the NFC harvester is further configured to charge the capacitor through the second switch to supply the load with an energy from the NFC harvester.

5. The power management unit of claim 4, wherein the CCU is further configured to enable the DC-DC converter to start boosting an output voltage from the TEG harvester.

6. The power management unit of claim 5, wherein when the output voltage from the DC-DC converter reaches a voltage level between the first switch and the second switch, the first switch is turned on and the load is supplied with an energy from the TEG harvester.

7. The power management unit of any preceding claim, further comprising a shunt clamp coupled in parallel with the capacitor and the load.

8. The power management unit of any preceding claim, further comprising a third switch coupled between the capacitor and the load.

9. The power management unit of any preceding claim, further comprising a rectifier coupled between the TEG harvester and the DC-DC converter.

10. The power management unit of any preceding claim, further comprising a second capacitor coupled between the TEG harvester and the DC-DC converter.

11. The power management unit of any preceding claim, wherein the power management unit does not include a battery.

12. The power management unit of any preceding claim, wherein the power management unit comprises the load.

13. A power management unit comprising:
a Thermoelectric Energy Generator (TEG) harvester;
a Near Field Communication (NFC) harvester;
a Direct Current (DC)-DC converter coupled between the TEG harvester and the NFC harvester;
a first switch and a second switch coupled between the DC-DC converter and the NFC harvester;
a central control unit (CCU) coupled between the first switch and the second switch and configured to control the DC-DC converter;
a capacitor coupled between the CCU, the first and second switches, and a load;
a third switch coupled between the capacitor and the load; and
a shunt clamp coupled in parallel with the capacitor and the load.

14. The power management unit of claim 13, wherein the NFC harvester is further configured to charge the capacitor through the second switch during a start-up event to activate the CCU, and wherein the second switch comprises a unidirectional switch.

15. The power management unit of claim 13 or 14, wherein the NFC harvester is further configured to charge the capacitor through the second switch to supply the load with an energy from the NFC harvester.
